# EUROPEAN PATENT SPECIFICATION

(11) **EP 3 914 238 B1**
(45) Date of publication and mention of the grant of the patent: **25.11.2026**
(21) Application number: 20701845.8
(22) Date of filing: 16.01.2020
(51) Int. Cl.: A61K 31/05, A61K 31/352, A61K 36/185, A61K 45/06, A61P 25/00, A61P 25/08

(54) **USE OF CBD FOR IMPROVING LANGUAGE IN A PATIENT WITH LENNOX-GASTAUT SYNDROME OR DRAVET SYNDROME**
VERWENDUNG VON CBD ZUR VERBESSERUNG DER SPRACHE BEI EINEM PATIENTEN MIT LENNOX-GASTAUT-SYNDROM ODER DRAVET-SYNDROM
UTILISATION DE CBD POUR AMÉLIORER LA LANGUE CHEZ UN PATIENT ATTEINT DU SYNDROME DE LENNOX-GASTAUT OU DU SYNDROME DE DRAVET

(30) Priority: 21.01.2019 GB 201900797
(43) Date of publication of application: 01.12.2021
(73) Proprietor: Jazz Pharmaceuticals Research UK Limited, Sittingbourne Kent ME9 8AG (GB)
(72) Inventor: GUY, Geoffrey, Sittingbourne, Kent ME9 8AG (GB); KNAPPERTZ, Volker, Sittingbourne, Kent ME9 8AG (GB); WHALLEY, Benjamin, Sittingbourne, Kent ME9 8AG (GB); WOOLLEY-ROBERTS, Marie, Sittingbourne, Kent ME9 8AG (GB)
(74) Representative: HGF
(86) International application number: PCT/GB2020/050090
(87) International publication number: WO 2020/152438

(56) References cited:
- WO-A1-2019/207319
- GB-A- 2 531 282
- GB-A- 2 542 155
- GB-A- 2 548 873
- RUTH GALLILY ET AL: "Overcoming the Bell-Shaped Dose-Response of Cannabidiol by Using <i>Cannabis</i> Extract Enriched in Cannabidiol", PHARMACOLOGY & PHARMACY, vol. 06, no. 02, 1 January 2015 (2015-01-01), United States, pages 75 - 85, XP055606394, ISSN: 2157-9423, DOI: 10.4236/pp.2015.62010

## Description

### FIELD OF THE INVENTION

The present invention relates to the use of a specific composition of cannabidiol (CBD) in the treatment of comorbidities associated with epilepsy. In one embodiment the comorbidities are surprisingly found to be improved patients where there is as absence of reduction in seizures.

The CBD used is in the form of a highly purified extract of cannabis such that the CBD is present at greater than 98% of the total extract (w/w) and the other components of the extract are characterised. In particular the cannabinoid tetrahydrocannabinol (THC) is present in an amount of from 0.01 to 0.1% (w/w).

### BACKGROUND TO THE INVENTION

Epilepsy occurs in approximately 1% of the population worldwide, (Thurman *et al*., 2011) of which 70% are able to adequately control their symptoms with the available existing anti-epileptic drugs (AED). However, 30% of this patient group, (Eadie *et al*., 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as *"failure of adequate trials of two tolerated and appropriately chosen and used AED schedules (whether as monotherapies or in combination) to achieve sustained seizure freedom"* (Kwan *et al*., 2009).

Individuals who develop epilepsy during the first few years of life are often difficult to treat and as such are often termed treatment-resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from, an investigation into the type of seizures that the patient is experiencing is undertaken. Clinical observations and electroencephalography (EEG) tests are conducted and the type(s) of seizures are classified according to the ILAE classification described below.

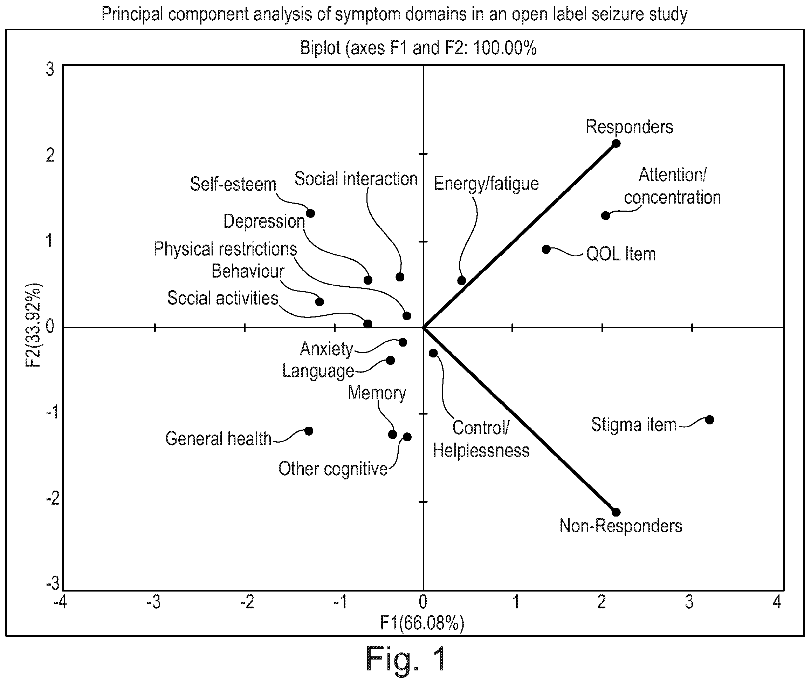

The International classification of seizure types proposed by the ILAE was adopted in 1981 and a revised proposal was published by the ILAE in 2010 and has not yet superseded the 1981 classification. Figure 1 is adapted from the 2010 proposal for revised terminology and includes the proposed changes to replace the terminology of partial with focal. In addition, the term "simple partial seizure" has been replaced by the term "focal seizure where awareness / responsiveness is not impaired" and the term "complex partial seizure" has been replaced by the term "focal seizure where awareness / consciousness is impaired".

Generalised seizures, where the seizure arises within and rapidly engages bilaterally distributed networks, can be split into six subtypes: Tonic-Clonic (grand mal) seizures; Absence (petit mal) Seizures; Clonic Seizures; Tonic Seizures; Atonic Seizures and Myoclonic Seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a Bilateral convulsive seizure, which is the proposed terminology to replace Secondary Generalised Seizures (generalized seizures that have evolved from focal seizures and are no longer remain localized).

Epileptic syndromes often present with many different types of seizure and identifying the types of seizure that a patient is suffering from is important as many of the standard AED's are targeted to treat or are only effective against a given seizure type / subtype.

One such childhood epilepsy is Dravet syndrome. Onset of Dravet syndrome almost always occurs during the first year of life with clonic and tonic-clonic seizures in previously healthy and developmentally normal infants (Dravet, 2011). Symptoms peak at about five months of age. Other seizures develop between one and four years of age such as prolonged focal dyscognitive seizures and brief absence seizures.

In diagnosing Dravet syndrome both focal and generalised seizures are considered to be mandatory, Dravet patients may also experience atypical absence seizures, myoclonic absence seizures, atonic seizures and non-convulsive status epilepticus.

Seizures progress to be frequent and treatment-resistant, meaning that the seizures do not respond well to treatment. They also tend to be prolonged, lasting more than 5 minutes. Prolonged seizures may lead to status epilepticus, which is a seizure that lasts more than 30 minutes, or seizures that occur in clusters, one after another.

Prognosis is poor and approximately 14% of children die during a seizure, because of infection, or suddenly due to uncertain causes, often because of the relentless neurological decline. Patients develop intellectual disability and life-long ongoing seizures. Intellectual impairment varies from severe in 50% patients, to moderate and mild intellectual disability each accounting for 25% of cases.

There are currently no FDA approved treatments specifically indicated for Dravet syndrome. The standard of care usually involves a combination of the following anticonvulsants: clobazam, clonazepam, levetiracetam, topiramate and valproic acid.

Stiripentol is approved in Europe for the treatment of Dravet syndrome in conjunction with clobazam and valproic acid. In the US, stiripentol was granted an Orphan Designation for the treatment of Dravet syndrome in 2008; however, the drug is not FDA approved.

Potent sodium channel blockers used to treat epilepsy actually increase seizure frequency in patients with Dravet Syndrome. The most common are phenytoin, carbamazepine, lamotrigine and rufinamide.

Management may also include a ketogenic diet, and physical and vagus nerve stimulation. In addition to anti-convulsive drugs, many patients with Dravet syndrome are treated with anti-psychotic drugs, stimulants, and drugs to treat insomnia.

Another such childhood epilepsy syndrome is Lennox-Gastaut syndrome (LGS). LGS is a severe form of epilepsy, where seizures usually begin before the age of 4. Seizure types, which vary among patients, include tonic (stiffening of the body, upward deviation of the eyes, dilation of the pupils, and altered respiratory patterns), atonic (brief loss of muscle tone and consciousness, causing abrupt falls), atypical absence (staring spells), and myoclonic (sudden muscle jerks). There may be periods of frequent seizures mixed with brief, relatively seizure-free periods.

Seizures in LGS are often described as "drop seizures". Such drop seizures are defined as an attack or spell (atonic, tonic or tonic-clonic) involving the entire body, trunk or head that led or could have led to a fall, injury, slumping in a chair or hitting the patient's head on a surface.

Most patients with LGS experience some degree of impaired intellectual functioning or information processing, along with developmental delays, and behavioural disturbances.

LGS can be caused by brain malformations, perinatal asphyxia, severe head injury, central nervous system infection and inherited degenerative or metabolic conditions. In 30-35% of cases, no cause can be found.

The first line treatment for drop seizures, including the treatment of drop seizures in patients with LGS, usually comprises a broad-spectrum AED, such as sodium valproate often in combination with rufinamide or lamotrigine. Other AEDs that may be considered include felbamate, clobazam and topiramate.

Comorbidities occur in many patients diagnosed with epilepsy or epilepsy syndromes.

A comorbidity is defined as the presence of one or more additional disorders that co-occur with a primary condition. There are multiple comorbid conditions associated with epilepsy. The 2007 NINDS Epilepsy Research Benchmarks included comorbidities as one of the benchmarks (Benchmarks Area III: Prevent, limit, and reverse the co-morbidities associated with epilepsy and its treatment) and the Institute of Medicine also identified epilepsy comorbidities in the IOM report on epilepsy.

Many conditions may be co-morbid with epilepsy however, commonly occurring comorbidities in epilepsy include psychiatric disorders, cognitive disorders, migraine, sleep disorders; cardiovascular, respiratory, inflammatory disorders and sudden unexpected death in epilepsy (SUDEP).

Some antiepileptic medications can have a negative effect on cognitive dysfunction, mood or behavior and should be used with caution in patients with existing comorbidities in these areas. AEDs can also cause behavioral adverse effects, and these effects may be more commonly seen in people with coexisting behavioral comorbidities.

A paper published in 2009 describes the following co-morbid conditions which have significantly higher rates in populations with epilepsy than those of the general population (Seidenberg *et al.* 2009).

Medical: musculoskeletal system disorders; gastrointestinal and digestive disorders; respiratory system disorders; chronic pain disorders; cerebrovascular accidents; migraine; neoplasia; arthritis; rheumatism; obesity; diabetes; infections; fractures and allergies.

Psychiatric: depression; anxiety; autism spectrum disorders; interictal dysphoric disorder; interictal behaviour syndrome and psychosis in epilepsy.

Cognitive: attention-deficit hyperactivity disorder; learning disability; mental retardation; Alzheimer's disease and dementia.

Rosenberg *et al.* 2017 describes a study where the quality of life (QOL) was measured in a care-giver-reported questionnaire of Quality of Life in Childhood Epilepsy (QOLCE). Paediatric patients with epilepsy were enrolled in a 12 weeks prospective open label study of CBD. Improvements were seen in areas of energy/fatigue; memory; other cognitive functions; control/helplessness; social interactions; behaviour and global quality of life. The group report that the changes in QOL were not correlated to changes in seizure frequency or adverse effects. The group also acknowledge the limitations associated with non-blinded studies lacking comparator groups in addition to the inherent bias associated with such questionnaires.

GB 2 531 282 A discloses CBD for use in the treatment of epilepsy, wherein the epilepsy is a treatment-resistant epilepsy, and wherein the epilepsy to be treated is characterised by atonic seizures. GB 2 542 155 A discloses CBD as an adjunct medicament in the treatment of a mental disorder where psychotic symptoms are being treated with a typical or an atypical antipsychotic, characterised in that the CBD is for use in augmenting the effect of the antipsychotic medicament. GB 2 548 873 A discloses CBD for use in the treatment of Sturge Weber syndrome.

R. Gallily, et al., Pharmacology & Pharmacy, 2015, 6, 75-85, studied in mice the anti-inflammatory and anti-nociceptive activities of standardized plant extracts derived from the *Cannabis sativa* L., clone 202, which is highly enriched in CBD and hardly contains any psychoactive ingredients. In contrast to purified CBD, the clone 202 extract, when given either intraperitoneally or orally, provided a clear correlation between the anti-inflammatory and anti-nociceptive responses and the dose, with increasing responses upon increasing doses.

The applicant has shown that the administration of a specific composition of CBD in patients with epilepsy has a significant impact on the treatment of certain comorbidities associated with epilepsy. Surprisingly it was found that certain comorbidities were improved in patients regardless of an improvement of seizure burden.

The areas found to be improved were attention and concentration; stigma item; general health; language and social activity. These improvements were seen in both open label studies and randomised controlled trials and as such provide robust evidence of such improvements in quality of life.

The CBD used is in the form of a highly purified extract of cannabis such that the CBD is present at greater than 98% of the total extract (w/w) and the other components of the extract are characterised. In particular the cannabinoid tetrahydrocannabinol (THC) is present in an amount of from 0.02 to 0.1% (w/w).

The co-pending application WO2019/207319 describes the surprising finding that a botanically derived purified CBD preparation which comprises minor amounts of the cannabinoids CBD-C1, CBDV, CBD-C4 and THC has an increased efficacy over a synthetic CBD which does not comprise minor amounts of cannabinoids.

These data are particularly surprising particularly given the fact that the concentration of CBD within the botanically derived purified CBD preparation and the synthetic preparation were the same.

The present invention demonstrates the ability of the same botanically derived purified CBD to improve certain comorbidities associated with epilepsy.

### BRIEF SUMMARY OF THE DISCLOSURE

In accordance with a first aspect of the present invention there is provided a cannabidiol (CBD) preparation for use in improving language in a patient with treatment resistant epilepsy (TRE), wherein the TRE is Lennox-Gastaut syndrome or Dravet syndrome, and wherein the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), wherein the preparation comprises about 0.01% to about 0.1% (w/w) THC based on total amount of cannabinoid in the preparation, and wherein the THC is present as a mixture of trans-THC and cis-THC.

Preferably the CBD comprises about 0.02% to about 0.05% (w/w) THC based on total amount of cannabinoid in the preparation.

In a further embodiment the mixture of trans-THC and cis-THC is present at a ratio of about 3.6:1 trans-THC:cis-THC.

More preferably the mixture of trans-THC and cis-THC is present at a ratio of about 0.8:1 trans-THC:cis-THC.

In a further embodiment the CBD preparation comprises about 0.1% to about 0.15% (w/w) CBD-C1 based on total amount of cannabinoid in the preparation.

In a further embodiment the CBD preparation comprises about 0.2% to about 0.8% (w/w) CBDV based on total amount of cannabinoid in the preparation.

In a further embodiment the CBD preparation comprises about 0.3% to about 0.4% (w/w) CBD-C4 based on total amount of cannabinoid in the preparation.

Preferably the dose of CBD is between 5 and 50 mg/kg/day.

Disclosed herein but not forming part of the invention is a method of treating quality of life associated with epilepsy comprising administering a cannabidiol (CBD) preparation for use in the treatment of quality of life domains associated with epilepsy wherein the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC cannabidiol (CBD) to a subject in need thereof.

Preferably the subject is a human. Alternatively, the subject is an animal. Preferably the animal is a dog.

### BRIEF DESCRIPTION OF THE DRAWINGS

Embodiments of the invention are further described hereinafter with reference to the accompanying drawings, in which:
Figure 1 shows the principal component analysis of symptom domains in an open label seizure study;
Figure 2 shows the principal component analysis of symptom domains in a randomized controlled trial; and
Figure 3 shows the correlation between seizure index ratio and cognitive performance in a rat model of epilepsy.

### DEFINITIONS

Definitions of some of the terms used to describe the invention are detailed below:

The cannabinoids described in the present application are listed below along with their standard abbreviations.

**Table 1. Cannabinoids and their abbreviations**

| | | |
|---|---|---|
| CBD | Cannabidiol | 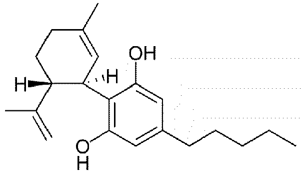 |
| THC | Tetrahydrocannabinol | 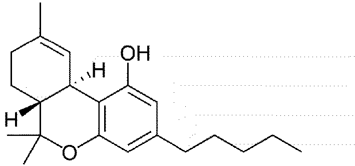 |
| CBDV | Cannabidivarin | 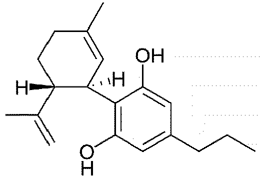 |
| CBD-C4 | Cannabidiol-C4 | 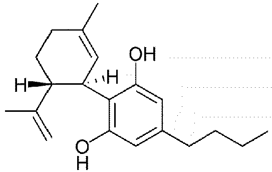 |
| CBD-C4 | Cannabidiol-C1 | 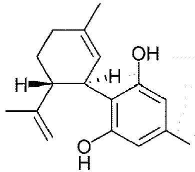 |

The table above is not exhaustive and merely details the cannabinoids which are identified in the present application for reference. So far over 60 different cannabinoids have been identified and these cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoid extracts" are defined as cannabinoids that have been extracted from the cannabis plant and purified to the extent that other cannabinoids and non-cannabinoid components that are co-extracted with the cannabinoids have been substantially removed, such that the highly purified cannabinoid is greater than or equal to 98% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Childhood epilepsy" refers to the many different syndromes and genetic mutations that can occur to cause epilepsy in childhood. Examples of some of these are as follows: Dravet Syndrome; Myoclonic-Absence Epilepsy; Lennox-Gastaut syndrome; Generalized Epilepsy of unknown origin; CDKL5 mutation; Aicardi syndrome; tuberous sclerosis complex; bilateral polymicrogyria; Dup15q; SNAP25; and febrile infection related epilepsy syndrome (FIRES); benign rolandic epilepsy; juvenile myoclonic epilepsy; infantile spasm (West syndrome); and Landau-Kleffner syndrome. The list above is non-exhaustive as many different childhood epilepsies exist.

"Comorbidities in epilepsy" refers to diseases or conditions that occur in addition to epilepsy. These include the following: Medical diseases or conditions: musculoskeletal system disorders; gastrointestinal and digestive disorders; respiratory system disorders; chronic pain disorders; cerebrovascular accidents; migraine; neoplasia; arthritis; rheumatism; obesity; diabetes; infections; fractures and allergies. Psychiatric diseases or conditions: depression; anxiety; autism spectrum disorders; interictal dysphoric disorder; interictal behaviour syndrome and psychosis in epilepsy. Cognitive diseases or conditions: attention-deficit hyperactivity disorder; learning disability; mental retardation; Alzheimer's disease and dementia.

"Quality of Life (QOL) Measures" are questionnaires or surveys undertaken by the patient or the patients care-giver which questions relate to how their condition affects their quality of life. These are used to monitor the impact of the disease on a patient's life and determine whether a treatment is enabling an improvement in such areas.

### DETAILED DESCRIPTION

### PREPARATION OF HIGHLY PURIFIED CBD EXTRACT

The following describes the production of the botanically derived purified CBD which has a known and constant composition was used in the Examples below.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 98% CBD. Although the CBD is highly purified because it is produced from a cannabis plant rather than synthetically there is a small amount of other cannabinoids which are co-produced and co-extracted with the CBD. Details of these cannabinoids and the quantities in which they are present in the medication are as follows:

| **Cannabinoid** | **Concentration** |
|---|---|
| CBDV | 0.2 - 0.8% (w/w) |
| CBD-C4 | 0.3 - 0.4% (w/w) |
| CBD-C1 | 0.1 - 0.15% (w/w) |
| THC | 0.01 - 0.1% (w/w) |

The CBD preparation comprises about 0.01% to about 0.1% THC based on total amount of cannabinoid in the preparation. In some embodiments, the CBD preparation comprises about 0.02% to about 0.05% THC based on total amount of cannabinoid in the preparation. In some embodiments, the CBD preparation comprises at least about 0.1% THC based on total amount of cannabinoid in the preparation. In some embodiments, the CBD preparation comprises at least about 0.02% THC based on total amount of cannabinoid in the preparation. In some embodiments, the THC comprises Δ9-THC.

The THC is present as a mixture of trans-THC and cis-THC. In some embodiments, the trans-THC and cis-THC are present at a ratio of about 5:1 (trans-THC:cis-THC). In some embodiments, the trans-THC and cis-THC are present at a ratio of about 3.5:1 (trans-THC:cis-THC). In some embodiments, the trans-THC and cis-THC are present at a ratio of about 2:1 (trans-THC:cis-THC). In some embodiments, the trans-THC and cis-THC are present at a ratio of about 1:1 (trans-THC:cis-THC). In some embodiments, the trans-THC and cis-THC are present at a ratio of about 0.8:1 (trans-THC:cis-THC).

In some embodiments the cis-THC is present as a mixture of (-)-cis-THC and (+)-cis-THC. In some embodiments, the (-)-cis-THC and (+)-cis-THC are present at a ratio of about 20:1 to 1:20 ((-)-cis-THC:(+)-cis-THC). In some embodiments, the (-)-cis-THC and (+)-cis-THC are present at a ratio of about 15:1 to 1:15 ((-)-cis-THC:(+)-cis-THC). In some embodiments, the (-)-cis-THC and (+)-cis-THC are present at a ratio of about 10:1 to 1:10 ((-)-cis-THC:(+)-cis-THC). In some embodiments, the (-)-cis-THC and (+)-cis-THC are present at a ratio of about 9:1 to 1:9 ((-)-cis-THC:(+)-cis-THC). In some embodiments, the (-)-cis-THC and (+)-cis-THC are present at a ratio of about 5:1 to 1:5 ((-)-cis-THC:(+)-cis-THC). In some embodiments, the (-)-cis-THC and (+)-cis-THC are present at a ratio of about 3:1 to 1:3 ((-)-cis-THC:(+)-cis-THC). In some embodiments, the (-)-cis-THC and (+)-cis-THC are present at a ratio of about 2:1 to 1:2 ((- )-cis-THC:(+)-cis-THC). In some embodiments, the (-)-cis-THC and (+)-cis-THC are present at a ratio of about 1:1 ((-)-cis-THC:(+)-cis-THC). In some embodiments, the (-)-cis-THC and (+)-cis-THC are present at a ratio of about 9:1 ((-)-cis-THC:(+)-cis-THC).

In some embodiments, the CBD preparation comprises one or more cannabinoids other than THC.

In some embodiments, the CBD preparation comprises cannabidivarin (CBDV). In some embodiments, the CBDV comprises the (-)- trans-CBDV isoform. In some embodiments, the CBD preparation comprises about 0.2% to about 0.8% CBDV based on total amount of cannabinoid in the preparation.

In some embodiments, the CBD preparation comprises CBD-C4 (CBD-C4). In some embodiments, the CBD-C4 comprises (-)-trans-CBD-C4 isoform. In some embodiments, the CBD preparation comprises about 0.3% to about 0.4% CBD-C4 based on total amount of cannabinoid in the preparation.

In some embodiments, the CBD preparation comprises CBD-C1 (CBD-C1). In some embodiments, the CBD-C1 comprises (-)- trans-CBD-C1 isoform. In some embodiments, the CBD preparation comprises about 0.1% to about 0.15% CBD-C1 based on total amount of cannabinoid in the preparation.

In some embodiments, at least a portion of at least one of the cannabinoids present in the CBD preparation is isolated from cannabis plant material. In some embodiments, at least a portion of the CBD present in the CBD preparation is isolated from cannabis plant material. In some embodiments, at least a portion of the THC present in the CBD preparation is isolated from cannabis plant material. In some embodiments, substantially all of at least one of the cannabinoids present in the CBD preparation is isolated from cannabis plant material. In some embodiments, substantially all the CBD present in the CBD preparation is isolated from cannabis plant material. In some embodiments, substantially all the THC present in the CBD preparation is isolated from cannabis plant material. In some embodiments, substantially all of the cannabinoids present in the CBD preparation are isolated from cannabis plant material. In some embodiments, the cannabis plant material is from a Cannabis sativa, Cannabis indica, or Cannabis ruderalis plant. In some embodiments, the cannabis plant is a high-CBD containing cannabis chemotype. In some embodiments, the cannabis plant is a high-CBD containing cannabis chemotype of Cannabis sativa L. In some embodiments, the cannabis plant material comprises about 5% to about 20% CBD based on total amount of cannabinoid in the preparation. In some embodiments, the cannabis plant material comprises about 10% to about 15% CBD based on total amount of cannabinoid in the preparation. In some embodiments, the cannabis plant material comprises trans-THC and cis-THC are present at a ratio of about 3.5:1 (trans-THC:cis-THC). In some embodiments, the cannabis plant material comprises trans-THC and cis-THC are present at a ratio of about 0.8:1 (trans-THC:cis-THC).

### EXAMPLE 1: PRINCIPAL COMPONENT ANALYSIS (PCA) OF OPEN LABEL STUDY DATA

Principal component analysis (PCA) is a statistical procedure that uses an orthogonal transformation to convert a set of observations of possibly correlated variables into a set of values of linearly uncorrelated variables called principal components.

This transformation is defined in such a way that the first principal component has the largest possible variance (accounts for as much of the variability in the data as possible), and each succeeding component in turn has the highest variance possible under the constraint that it is orthogonal to the preceding components.

PCA is a useful tool to visualise relatedness between populations of data and was used in the present example to determine the symptom domains that were improved during an open label study of cannabidiol (CBD) in patients with treatment resistant epilepsy.

### Materials and Methods

Children and young adults with severe, childhood onset treatment-resistant epilepsy (TRE) were tested with a highly purified extract of cannabidiol (CBD) obtained from a cannabis plant. The participants in the study were part of an expanded access compassionate use program for CBD.

All patients entered a baseline period of 4 weeks when parents/caregivers kept prospective seizure diaries, noting all countable motor seizure types.

The patients then received a highly purified CBD extract (greater than 98% CBD w/w) in sesame oil, of known and constant composition, at a dose of 5 mg/kg/day in addition to their baseline anti-epileptic drug (AED) regimen.

The daily dose was gradually increased by 2 to 5mg/kg increments until intolerance occurred or a maximum dose of 50 mg/kg/day was achieved.

Patients were seen at regular intervals of 2-4 weeks. Laboratory testing for hematologic, liver, kidney function, and concomitant AED levels was performed at baseline, and at regular intervals throughout the 12 weeks of the study.

The Quality of Life in Childhood Epilepsy (QOLCE) survey was used to measure multiple QOL domains. Caregivers completed the survey at baseline and after the 12 weeks of treatment. The QOLCE questionnaire assessed 91 items divided into five sub-domains: physical function; cognitive function; emotional well-being; social function and behaviour. General health was also recorded.

PCA was undertaken on the QOLCE data collected and the reduction in seizures to determine whether participants experienced a reduction in certain areas of QOL as a result of a reduction in seizures or whether there was a reduction in QOL domains independent of the response to the study drug by way of seizure reduction.

### Results

Figure 1 details the principal component analysis of the data obtained from the QOLCE survey.

QOL domains that are plotted to the right of the zero point on the F1 axis are those domains which showed an improvement over baseline. Conversely the QOL domains plotted to the left of the zero point on the F1 axis are those domains that did not improve over the baseline measures.

The QOL domains that are plotted above the zero point on the F2 axis are those domains that improved in participants that experienced a reduction in seizures and the QOL domains plotted below the zero point on the F2 axis are the areas which improved despite a reduction in seizures.

Table 2 below additionally details the percentage contributions of the observations and Table 3 provide the squared cosines of the observations.

**Table 2: Percentage contribution of the observations**

| | **F1** | **F2** |
|---|---|---|
| **Observation** | **(%)** | **(%)** |
| Physical restrictions | 0.1882 | 0.2021 |
| Energy / fatigue | 0.8195 | 2.774 |
| Attention / concentration | 18.9846 | 15.4435 |
| Memory | 0.5998 | 13.6648 |
| Language | 0.6912 | 1.2598 |
| Other cognitive | 0.1950 | 14.4822 |
| Depression | 1.9285 | 2.8150 |
| Anxiety | 0.2729 | 0.2411 |
| Control / helplessness | 0.0450 | 0.7555 |
| Self-esteem | 7.7012 | 16.1366 |
| Social interaction | 0.3579 | 3.1636 |
| Social activities | 1.9243 | 0.0059 |
| Stigma item | 47.2746 | 10.1364 |
| Behaviour | 6.5082 | 0.8793 |
| General health | 7.8569 | 13.0117 |
| QOL item | 4.6521 | 5.0251 |

**Table 3: Squared cosines of the observations**

| **Observation** | **F1** | **F2** |
|---|---|---|
| Physical restrictions | 0.6447 | 0.3553 |
| Energy / fatigue | 0.3650 | 0.6350 |
| Attention / concentration | 0.7055 | 0.2945 |
| Memory | 0.0788 | 0.9212 |
| Language | 0.5167 | 0.4833 |
| Other cognitive | 0.0256 | 0.9744 |
| Depression | 0.5715 | 0.4283 |
| Anxiety | 0.6880 | 0.3120 |
| Control / helplessness | 0.1040 | 0.8960 |
| Self-esteem | 0.4818 | 0.5182 |
| Social interaction | 0.1806 | 0.8194 |
| Social activities | 0.9984 | 0.0016 |
| Stigma item | 0.9009 | 0.0991 |
| Behaviour | 0.9352 | 0.0648 |
| General health | 0.5405 | 0.4595 |
| QOL item | 0.6433 | 0.3567 |

These data demonstrate that there were improvements seen in several areas associated with quality of life (to the right of the zero F1 axis). These areas were: attention and concentration; QOL item; energy/fatigue; control/helplessness and stigma item.

In the areas of control/helplessness and stigma item improvement was surprisingly observed without an improvement of seizures (bottom right area of the biplot).

### Conclusions

There was found to be an improvement several areas associated with quality of life. The improvements in these key areas will enable patients to enjoy a better quality of life. The fact that improvements were seen in several of these areas irrespective of a reduction of seizures means that despite the medication being ineffective in reduction of seizures in these patients they enjoyed an improvement in quality of life. This is particularly surprising given that these patients were treatment resistant and had tried and failed at least three anti-epileptic drugs prior to the study.

### EXAMPLE 2: PRINCIPAL COMPONENT ANALYSIS (PCA) OF RANDOMISED CONTROLLED TRIAL POOLED STUDY DATA

As described in Example 1 above PCA is a useful tool to scrutinise data which have several variables. A similar analysis was undertaken on the data produced using data pooled from three randomised controlled clinical trials on children and young adults with treatment resistant epilepsy associated with Dravet syndrome (one trial) and Lennox-Gastaut syndrome (LGS) (2 trials).

### Materials and Methods

In the two placebo-controlled studies of CBD as a treatment for seizures associated with LGS, cannabidiol (CBD) was used as add-on treatment in patients who were defined as treatment-resistant. Patients had previously tried and stopped using a median of 6 AEDs and were being maintained on a median of 3 AEDs.

The first study was a 1:1 randomised, double-blind, 14-week comparison of cannabidiol oral solution (CBD-OS) versus placebo. The treatment period consisted of a two-week titration period followed by a 12-week maintenance period. The treatment period was followed by a 10-day taper period and a four-week follow-up period. The study aimed to determine the efficacy, safety and tolerability of 20 mg/kg/day cannabidiol compared with placebo.

The second study was a 1:1:1 randomised, double-blind, 14-week comparison of two dose levels of cannabidiol (10 mg/kg/day and 20 mg/kg/day) versus placebo. The treatment period consisted of a two-week titration period followed by a 12-week maintenance period. The treatment period was followed by a 10-day taper period and a four-week follow-up period. The study aimed to determine the efficacy, safety and tolerability of two dose levels of CBD-OS compared with placebo. Patients in the placebo group were split into two equivalent cohorts; half receiving 10 mg/kg/day dosing volumes and half receiving 20 mg/kg/day dosing volumes.

In the placebo-controlled studies of CBD as a treatment for seizures associated with Dravet syndrome, CBD was used as add-on treatment in patients who were defined as treatment-resistant. The study was a 1:1 randomised, double-blind, 14-week comparison of cannabidiol oral solution (CBD-OS) versus placebo. The treatment period consisted of a two-week titration period followed by a 12-week maintenance period. The treatment period was followed by a 10-day taper period and a four-week follow-up period. The study aimed to determine the efficacy, safety and tolerability of 20 mg/kg/day cannabidiol compared with placebo.

The Quality of Life in Childhood Epilepsy (QOLCE) survey was used to measure multiple QOL domains. Caregivers completed the survey at baseline and after the 12 weeks of treatment. The QOLCE questionnaire assessed 91 items divided into five sub-domains: physical function; cognitive function; emotional well-being; social function and behaviour. General health was also recorded.

### RESULTS

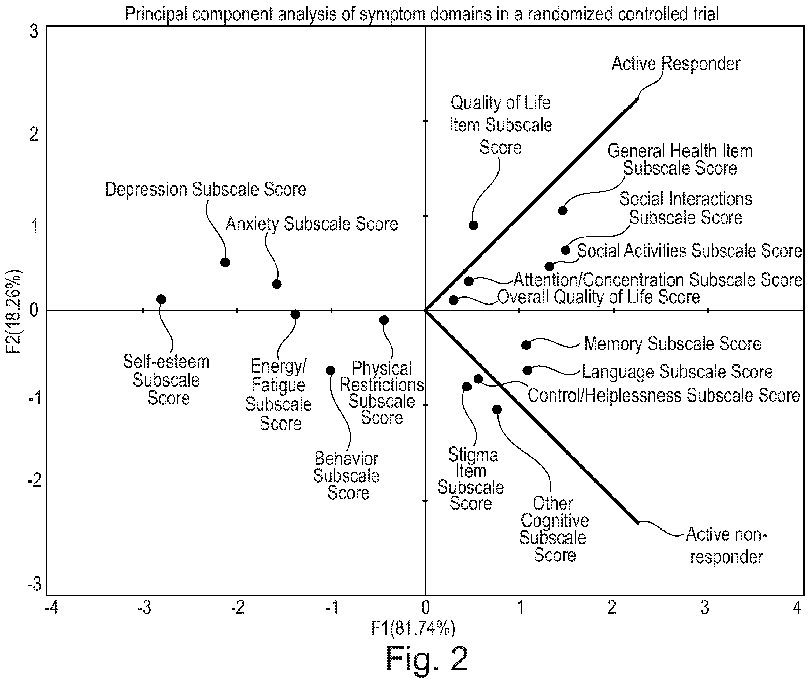

Figure 2 details the principal component analysis of the data obtained from the QOLCE survey.

As described in Example 1 the areas which showed an improvement in QOL domains are plotted to the right of the zero F1 axis and the areas where there was additionally a reduction in seizures are found above the zero point of the F2 axis.

Table 4 below additionally details the percentage contributions of the observations and Table 5 provide the squared cosines of the observations.

**Table 4: Percentage contribution of the observations**

| **Observation** | **F1** | **F2** |
|---|---|---|
| | **(%)** | **(%)** |
| Physical restrictions | 0.7253 | 0.1760 |
| Energy / fatigue | 6.8776 | 0.0135 |
| Attention / concentration | 0.7377 | 1.5619 |
| Memory | 0.5998 | 13.6648 |
| Language | 4.0924 | 6.3268 |
| Other cognitive | 2.0541 | 17.7509 |
| Depression | 16.2731 | 4.1063 |
| Anxiety | 8.9890 | 1.1755 |
| Control / helplessness | 1.1084 | 8.4404 |
| Self-esteem | 28.1650 | 0.1912 |
| Social interaction | 7.7974 | 6.5354 |
| Social activities | 6.0637 | 3.3786 |
| Stigma item | 0.6810 | 10.4914 |
| Behaviour | 6.5082 | 0.8793 |
| General health | 7.4459 | 18.1100 |
| QOL item | 0.9308 | 12.9483 |

**Table 5: Squared cosines of the observations**

| **Observation** | **F1** | **F2** |
|---|---|---|
| Physical restrictions | 0.6447 | 0.3553 |
| Energy / fatigue | 0.3650 | 0.6350 |
| Attention / concentration | 0.6790 | 0.3210 |
| Memory | 0.0788 | 0.9212 |
| Language | 0.7434 | 0.2566 |
| Other cognitive | 0.3413 | 0.6587 |
| Depression | 0.5715 | 0.4283 |
| Anxiety | 0.6880 | 0.3120 |
| Control / helplessness | 0.3703 | 0.6297 |
| Self-esteem | 0.4818 | 0.5182 |
| Social interaction | 0.8423 | 0.1577 |
| Social activities | 0.8893 | 0.1107 |
| Stigma item | 0.2252 | 0.7748 |
| Behaviour | 0.7195 | 0.2805 |
| General health | 0.6480 | 0.3520 |
| QOL item | 0.2435 | 0.7565 |

As can be seen improvements were seen in additional areas associated with quality of life compared to Example 1. These areas were quality of life item; general health; social interactions; social activities; attention/concentration; overall quality of life; memory; language; control/helplessness; stigma item and cognition.

Again, surprisingly some areas of QOL improved despite the patient not experiencing an improvement in their seizure burden. Areas where such improvement was observed were: memory; language; control/helplessness; stigma item and cognition.

### Conclusions

As suggested by the expanded access data described within Example 1 these data from a randomised controlled clinical trial of CBD in patients with Dravet syndrome and Lennox-Gastaut syndrome there was improvement in particular QOL domains exclusive of seizure reduction.

In patients that suffer with epilepsy it is often not the seizure burden that is the most devastating part of the disease. A decrease in certain QOL areas such as language and memory can be distressing to patients and their carers suffering from epilepsy syndromes. An improvement in these domains enables patients and their carers to live a more normal life despite still experiencing seizures.

### EXAMPLE 3: BIOSTATISTICAL ANALYSIS OF RANDOMISED CONTROLLED TRIAL LENNOX-GASTAUT STUDY DATA

Biostatistical analysis is the application of statistics to a wide range of topics in biology, in particular medical biostatistics, which is exclusively concerned with medicine and health.

The following example uses biostatistical analysis to look for trends in QOL data collected in a randomised controlled trial of cannabidiol in patients with Lennox-Gastaut syndrome.

### Methods

This study was a 1:1 randomised, double-blind, 14-week comparison of cannabidiol oral solution (CBD-OS) versus placebo. The treatment period consisted of a two-week titration period followed by a 12-week maintenance period. The treatment period was followed by a 10-day taper period and a four-week follow-up period. The study aimed to determine the efficacy, safety and tolerability of 20 mg/kg/day cannabidiol compared with placebo.

The Quality of Life in Childhood Epilepsy (QOLCE) survey was used to measure multiple QOL domains. Caregivers completed the survey at baseline and after the 12 weeks of treatment. The QOLCE questionnaire assessed 91 items divided into five sub-domains: physical function; cognitive function; emotional well-being; social function and behaviour. General health was also recorded.

### Results

Table 6 below details the areas where there no change (indicated by ↔) or a difference of greater than 10 points between medians (indicated by ↑), and whether this change was statistically significant (indicated by *).

**Table 6. Biostatistical analysis of QOL subscale scores versus placebo**

| **QOL subscale** | **Biostatistical analysis** |
|---|---|
| Attention / concentration | ↔ |
| Other cognitive | * |
| Anxiety | ↔ |
| Depression | ↔ |
| Memory | ↑* |
| Language | ↑* |
| Energy / fatigue | ↔ |
| Social interaction | ↑ |
| Social activities | ↔ |
| Control / helplessness | ↔ |
| Behaviour | ↔ |
| Physical restriction | ↔ |
| Self-esteem | ↔ |
| Stigma item | ↔ |
| General health | ↔ |

As can be seen there was a significant increase above baseline in the QOL domains of memory and language. This is consistent with the areas that were found to be improved in the pooled data analysis in Example 2.

Interestingly there were no domains where there was a decrease in quality of life.

### Conclusions:

The data presented in this Example supports the improvement in QOL areas of language and memory. This analysis of a smaller set of data provides strong supporting evidence of the improvement of specific QOL domains in patients with Lennox-Gastaut syndrome.

### EXAMPLE 4: CASE STUDY ANALYSIS OF OPEN LABEL STUDY DATA

Examples 1 to 3 detail the improvement in specific quality of life domains in patients with Dravet syndrome and Lennox-Gastaut syndrome.

The present example provides further data on additional epilepsy syndromes which were studied as part of the expanded access programme described in Example 1.

### Methods

Children and young adults with severe, childhood onset treatment-resistant epilepsy (TRE) were tested with a highly purified extract of cannabidiol (CBD) obtained from a cannabis plant. The participants in the study were part of an expanded access compassionate use program for CBD.

All patients entered a baseline period of 4 weeks when parents/caregivers kept prospective seizure diaries, noting all countable motor seizure types.

The patients then received a highly purified CBD extract (greater than 98% CBD w/w) in sesame oil, of known and constant composition, at a dose of 5 mg/kg/day in addition to their baseline anti-epileptic drug (AED) regimen.

The daily dose was gradually increased by 2 to 5mg/kg increments until intolerance occurred or a maximum dose of 50 mg/kg/day was achieved.

Patients were seen at regular intervals of 2-4 weeks. Laboratory testing for hematologic, liver, kidney function, and concomitant AED levels was performed at baseline, and at regular intervals throughout the 12 weeks of the study.

Quality of life was recorded either by physician, patient / caregiver or both and denoted by the measures of very much worse; much worse; worse; no change; slightly improved; much improved or very much improved at each visit.

### Results

Table 7 below details the results obtained at the end of the study period (after between 12 and 108 weeks) from patients suffering from epilepsy syndromes in an open label study.

**Table 7. QOL measures recorded in patients with epilepsy syndrome as part of an open label study**

| **Phenotype** | **Patient ID** | **Very much worse** | **Much worse** | **Slightly worse** | **No Change** | **Slightly improved** | **Much improved** | **Very much improved** |
|---|---|---|---|---|---|---|---|---|
| Tuberous sclerosis | FLAM-25 | | | | ✔ | | | |
| Tuberous sclerosis | LCH-22 | | | | | | | ✔ |
| CDKL5 | WIL-09 | | | | | | ✔ | |
| CDKL5 | 245-08 | | | | | | | ✔ |
| CDKL5 | 245-11 | | | | | | | ✔ |
| CDKL5 | 245-37 | | | | | | | ✔ |
| CDKL5 | 245-38 | | | | | ✔ | | |
| CDKL5 | 245-45 | | | | | ✔ | | |
| CDKL5 | 245-47 | | | | | | | ✔ |
| CDKL5 | 245-48 | | | | | | | ✔ |
| CDKL5 | 245-56 | | | ✔ | | | | |
| CDKL5 | LCH-21 | | | | | | | ✔ |
| Sturge Weber | WIL-02 | | | | | | ✔ | |
| Doose | WIL-05 | | | | | | ✔ | |
| Doose | FIL-14 | | | | | | ✔ | |
| Doose | FIL-09 | | | | | | | ✔ |
| Angelman | WIL-10 | | | | | | ✔ | |
| SNAP25 | 245-01 | | | | | | | ✔ |
| FIRES | 245-03 | | | | | | | ✔ |
| FIRES | LCH-18 | | | | | | | ✔ |
| FIRES | MAR-E1 | | ✔ | | | | | |
| FIRES | MAR-E2 | | | | | | | ✔ |
| DUP15Q | 245-06 | | | | | | ✔ | |
| DUP15Q | 245-32 | | | | | | ✔ | |
| DUP15Q | 245-40 | | | | | | ✔ | |
| DUP15Q | 245-42 | | | | | | ✔ | |
| DUP15Q | 245-43 | | | | | ✔ | | |
| Aicardi | 245-12 | | | | | | | ✔ |
| Aicardi | 245-28 | | | | | | ✔ | |
| Aicardi | 245-57 | | | | | | ✔ | |
| Aicardi | FIL-01 | | | | | | ✔ | |
| Aicardi | FIL-25 | | | ✔ | | | | |
| Aicardi | LCH-04 | | | | | | | ✔ |
| Aicardi | LCH-08 | | | | ✔ | | | |
| Aicardi | LCH-12 | | | | | | | ✔ |
| Aicardi | PAT-10 | | | | | | | ✔ |
| Aicardi | PAT-1 1 | | | | | | | ✔ |
| Aicardi | PAT-26 | | | | | | ✔ | |
| Aicardi | PAT-21 | | | | | | | ✔ |

As is observed in Table 7 there was an observable improvement in quality of life in patients diagnosed with tuberous sclerosis; CDKL5; Sturge Weber; Doose syndrome; Angelman syndrome; SNAP25; Febrile infection related epilepsy syndrome (FIRES); DUP15Q and Aicardi syndrome.

In the 39 patients for which data were recorded 34 provided a response which was positive (slightly improved; much improved or very much improved). Such data, although collected via open label means and as such open to bias, provide evidence that there is an improvement in quality of life within additional epilepsy syndromes aside from the more thoroughly studied Dravet syndrome and Lennox-Gastaut syndrome.

### Conclusions

The data presented in the Example suggests that improvements in quality of life measures are not restricted to particular epilepsy syndrome and are seen within patient populations suffering from various different childhood onset treatment resistant epilepsy syndromes.

### EXAMPLE 5: EFFICACY OF CANNABIDIOL IN COGNITIVE PERFORMANCE IN RATS

It has been shown previously that CBD could restore the deteriorated cognitive performances of rats exhibiting spontaneous recurrent seizures. However, CBD also improved the seizure ratio of these animals.

In order to understand if the improvement of the cognitive performances is just a consequence of the reduction of seizures severity by CBD, the correlation between seizure score and the behavioural performances was assessed via the Pearson correlation coefficient in the present Example.

### Methods

### INDUCTION OF EPILEPSY

The reduced intensity, status epilepticus (RISE) form of lithium-pilocarpine induced epilepsy in male Wistar rats (Harlan Envigo, UK; age: P21-28; weight >70g) was used.

The animals were maintained in 12h:12h dark:light cycle, a room temperature of 21°C and humidity of 50±10 %, with ad libitum access to food and water throughout the study period. Only animals that were classified as epileptic within 4-8 weeks from the day of induction were used in the present study to minimise any age-related variability.

### STUDY DESIGN

In order to understand if it exists a correlation between seizure burden and cognitive performances, as well as the influence of CBD on this relationship, 20 epileptic rats were divided into two groups where 10 animals were treated with 200 mg/kg CBD in drinking water for 6 weeks and 10 animals received only vehicle (3.5% Kolliphor^{®} HS, Sigma-Aldrich, Poole, UK) for the entire period of study.

A group of 10 vehicle treated healthy rats was also added. The drug/vehicle treatment was continued during the behavioural experiments (weeks 7 and 8 of drug treatment).

### DRUGS AND CHEMICALS

Highly purified CBD (>98% w/w) of botanical origin were used in the experiments.

### VIDEO MONITORING AND BEHAVIOURAL ASSESSMENT OF SEIZURES

All animals were video monitored 24 hours a day for the entire 6-week period of the study. Twenty CCTV cameras (TP-101BK, Topica, Taiwan) were established and connected to a PC, with video footage recorded using Zoneminder (v1.2.3; Triornis Ltd., Bristol, UK) software.

The video footage obtained during the light phase (07:00 to 19:00) from initial 4 days (at the beginning of treatment) and final 4 days (before beginning of the behavioural experiments) were coded offline to analyse rat behaviour.

A blinded independent researcher was trained to identify and code convulsive behaviours using a modified Racine scale. Scores of ≥ 3 were recorded and included in the results as these reflected clearly identifiable motor convulsion.

Seizure index of an animal was thereafter calculated by multiplying each severity score (in Racine scale) with the corresponding frequencies (number of occurrences) during the observation period.

The seizure index ratio was calculated from the seizure index by using the formula: Seizure ratio = (the mean seizure index in final bin) / (mean seizure index in first bin). A higher seizure ratio therefore indicates the worsening of the disease.

### COGNITIVE FUNCTION ASSESSMENT

A hole-board apparatus made of Plexiglas (70×70×45 cm), mounted on a table of 72 cm above the floor level was used to assess the cognitive function of the animals. The apparatus consisted of equally spaced 16 holes on its floor, each of 2.5 cm in diameter. Each rat was placed in the centre of the hole-board and allowed to freely explore the apparatus for 10 min.

Four accessible baits were kept in four holes, randomly selected for each animal, but kept constant across all test days. Olfactory cues were nullified by placing inaccessible baits in all other holes.

Prior to onset of testing, animals were habituated to the hole-board for four days (two days without bait to let the animals used to the test arena followed by two days with bait to habituate the animals to the rules of the task). Animals were food deprived for 4-5 hours on test days before beginning the test to enhance motivation to complete the task.

Testing was conducted for three consecutive days with animals performing five trials each day. Head dips were recorded by a video camera for later analysis. A head dip was scored when the head was introduced into a hole at least to eye level of the rodent.

Errors consisted of head-dipping a hole that was never baited (reference-memory error) or re-dipping a hole that had been baited (working-memory error). The total number of reference memory errors and working memory errors over the three days of test an animal made was used as a measure of cognitive performance. The mean number of reference and working memory errors over 15 trials was calculated for each animal.

### STATISTICAL ANALYSIS

The linear correlation between the seizure index and the number of reference and working memory errors was assessed for each animal using the Pearson correlation coefficient which has a value between +1 and -1, where +1 is a total positive correlation and -1 is a total negative correlation.

Note that the correlation reflects the non-linearity and direction of a linear relationship, but not the slope of that relationship, nor many aspects of nonlinear relationships. In all cases p<0.05 was considered significant.

### Results

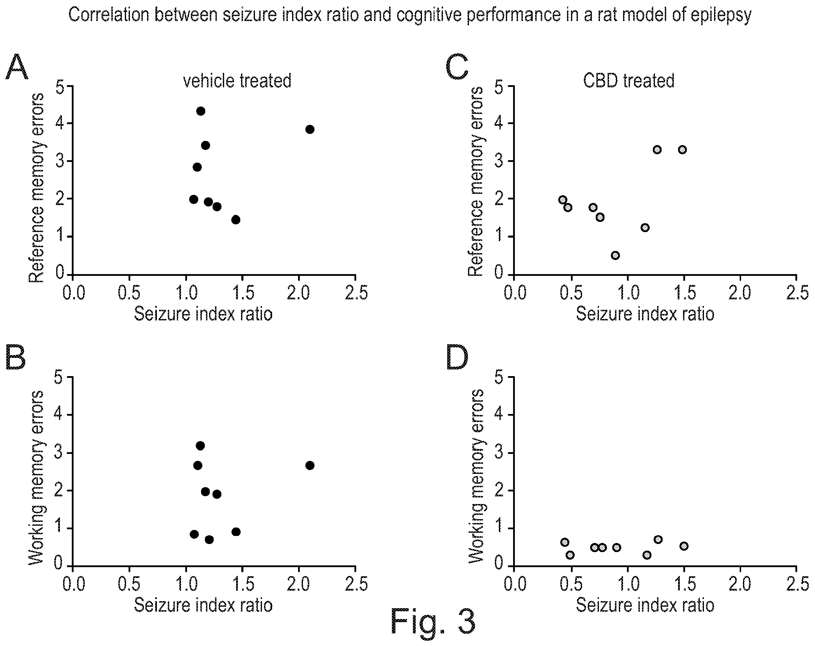

Figure 3 details the correlation between seizure index ratio and cognitive performances in the holeboard test.

In graph A there was no significant correlation between seizure index ratio and number of reference memory errors was found in vehicle treated rats (R2=0.05533, p=0.05749, n=8).

In graph B the seizure index ratio was not significantly correlated to the number of working memory errors in vehicle treated rats (R2=0.03884, p=0.6399, n=8).

In graph C the number of reference memory errors was not significantly correlated to the seizure index ration in CBD treated rats (R2=0.2486, p=0.2085, n=8).

In graph D there was no significant correlation between working memory errors and seizure index ratio in CBD treated animals (R2=0.04755, p=0.6039, n=8).

### Conclusion

The correlation analysis showed that no linear relationship existed between the seizure burden and cognitive performances exhibited by both vehicle and CBD treated RISE-SRS rats.

It suggests the beneficial effects of CBD on seizure burden and cognitive performances are independent of one another.

### Overall conclusion

Taken together, the data from the five examples presented herein provide evidence of the efficacy of highly purified CBD of botanical origin in improvements in quality of life.

In particular, improvements were seen in specific QOL domains including attention/concentration; stigma item; general health; language and social activity.

Such improvements were seen to be observed independent of reduction in seizures which suggests that the benefits were actual improvements in these specific areas rather than them being improved as a result of the patient suffering from less seizures.

## Claims

1. A Cannabidiol (CBD) preparation for use in improving language in a patient with treatment resistant epilepsy (TRE), wherein the TRE is Lennox-Gastaut syndrome or Dravet syndrome, and wherein the CBD preparation comprises greater than or equal to 98% (w/w) CBD and less than or equal to 2% (w/w) other cannabinoids, wherein the less than or equal to 2% (w/w) other cannabinoids comprises the cannabinoids THC; cannabidiol-C1 (CBD-C1); cannabidivarin (CBDV); and cannabidiol-C4 (CBD-C4), wherein the preparation comprises about 0.01% to about 0.1% (w/w) THC based on total amount of cannabinoid in the preparation, and wherein the THC is present as a mixture of trans-THC and cis-THC.

2. A CBD preparation for use according to claim 1, wherein the preparation comprises about 0.02% to about 0.05% (w/w) THC based on total amount of cannabinoid in the preparation.

3. A CBD preparation for use according to any of the preceding claims, wherein the preparation further comprises about 0.1% to about 0.15% (w/w) CBD-C1 based on total amount of cannabinoid in the preparation.

4. A CBD preparation for use according to any of the preceding claims, wherein the preparation further comprises about 0.2% to about 0.8% (w/w) CBDV based on total amount of cannabinoid in the preparation.

5. A CBD preparation for use according to any of the preceding claims, wherein the preparation further comprises about 0.3% to about 0.4% (w/w) CBD-C4 based on total amount of cannabinoid in the preparation.

6. A CBD preparation for use according to any of the preceding claims, wherein the dose of CBD is between 5 and 50 mg/kg/day.

## Patentansprüche

1. Cannabidiol(CBD-)Zubereitung zur Verwendung bei der Verbesserung der Sprache bei einem Patienten mit behandlungsresistenter Epilepsie (TRE), wobei die TRE Lennox-Gastaut-Syndrom oder Dravet-Syndrom ist und wobei die CBD-Zubereitung mehr als oder gleich 98 % (w/w) CBD und weniger als oder gleich 2 % (w/w) andere Cannabinoide umfasst, wobei die weniger als oder gleich 2 % (w/w) anderen Cannabinoide die Cannabinoide THC; Cannabidiol-C1 (CBD-C1); Cannabidivarin (CBDV); und Cannabidiol-C4 (CBD-C4) umfassen, wobei die Zubereitung etwa 0,01 % bis etwa 0,1 % (w/w) THC basierend auf Gesamtmenge an Cannabinoid in der Zubereitung umfasst und wobei das THC als Gemisch aus trans-THC und cis-THC vorhanden ist.

2. CBD-Zubereitung zur Verwendung nach Anspruch 1, wobei die Zubereitung etwa 0,02 % bis etwa 0,05 % (w/w) THC basierend auf Gesamtmenge an Cannabinoid in der Zubereitung umfasst.

3. CBD-Zubereitung zur Verwendung nach einem der vorhergehenden Ansprüche, wobei die Zubereitung ferner etwa 0,1 % bis etwa 0,15 % (w/w) CBD-C1 basierend auf Gesamtmenge an Cannabinoid in der Zubereitung umfasst.

4. CBD-Zubereitung zur Verwendung nach einem der vorhergehenden Ansprüche, wobei die Zubereitung ferner etwa 0,2 % bis etwa 0,8 % (w/w) CBDV basierend auf Gesamtmenge an Cannabinoid in der Zubereitung umfasst.

5. CBD-Zubereitung zur Verwendung nach einem der vorhergehenden Ansprüche, wobei die Zubereitung ferner etwa 0,3 % bis etwa 0,4 % (w/w) CBD-C4 basierend auf Gesamtmenge an Cannabinoid in der Zubereitung umfasst.

6. CBD-Zubereitung zur Verwendung nach einem der vorhergehenden Ansprüche, wobei die Dosis von CBD zwischen 5 und 50 mg/kg/Tag ist.

## Revendications

1. Préparation de cannabidiol (CBD) destinée à être utilisée pour améliorer le langage chez un patient atteint d'épilepsie résistante au traitement (TRE), dans laquelle la TRE est le syndrome de Lennox-Gastaut ou le syndrome de Dravet, et dans laquelle la préparation de CBD comprend au moins 98 % (p/p) de CBD et au plus 2 % (p/p) d'autres cannabinoïdes, dans laquelle les autres cannabinoïdes présents à au plus 2 % (p/p) comprennent les cannabinoïdes THC ; cannabidiol-C1 (CBD-C1) ; cannabidivarine (CBDV) ; et cannabidiol-C4 (CBD-C4), dans laquelle la préparation comprend environ 0,01 % à environ 0,1 % (p/p) de THC sur la base de la quantité totale de cannabinoïdes dans la préparation, et dans laquelle le THC est présent sous forme d'un mélange de trans-THC et de cis-THC.

2. Préparation de CBD destinée à être utilisée selon la revendication 1, dans laquelle la préparation comprend environ 0,02 % à environ 0,05 % (p/p) de THC sur la base de la quantité totale de cannabinoïdes dans la préparation.

3. Préparation de CBD destinée à être utilisée selon l'une quelconque des revendications précédentes, dans laquelle la préparation comprend en outre environ 0,1 % à environ 0,15 % (p/p) de CBD-C1 sur la base de la quantité totale de cannabinoïdes dans la préparation.

4. Préparation de CBD destinée à être utilisée selon l'une quelconque des revendications précédentes, dans laquelle la préparation comprend en outre environ 0,2 % à environ 0,8 % (p/p) de CBDV sur la base de la quantité totale de cannabinoïdes dans la préparation.

5. Préparation de CBD destinée à être utilisée selon l'une quelconque des revendications précédentes, dans laquelle la préparation comprend en outre environ 0,3 % à environ 0,4 % (p/p) de CBD-C4 sur la base de la quantité totale de cannabinoïdes dans la préparation.

6. Préparation de CBD destinée à être utilisée selon l'une quelconque des revendications précédentes, dans laquelle la dose de CBD est comprise entre 5 et 50 mg/kg/jour.
